# EUROPEAN PATENT APPLICATION

(11) **EP 4 844 001 A1**
(43) Date of publication of application: **25.11.2026**
(21) Application number: 25192146.6
(22) Date of filing: 28.07.2025
(51) Int. Cl.: C07K 14/72, C07K 7/06, G01N 21/552

(54) **PEPTIDE HAVING AN AFFINITY FOR A FP-RECEPTOR, DETECTION DEVICE AND USE**

(30) Priority: 21.05.2025 EP 25177904
(71) Applicant: Hochschule Kaiserslautern, 67659 Kaiserslautern (DE)
(72) Inventor: Tarasov, Alexey, 67659 Kaiserslautern (DE); Bufe, Bernd, 67659 Kaiserslautern (DE); Groß, Peter, 67659 Kaiserslautern (DE); Hasnain, Ahmar, 67659 Kaiserslautern (DE); Heilmann, Heiko, 67659 Kaiserslautern (DE)
(74) Representative: Durm Patentanwälte PartG mbB

(57) **Abstract**

The present invention relates to a peptide (1) having an affinity for a FP-receptor (9), a substrate (2) comprising the peptide (1), a process for immobilizing the peptide (1) to a substrate (2) and a process for detecting the presence of a cell (8) having a FP-receptor (9). The present invention further relates to a use of the peptide (1) for immobilizing a cell (8) having a FP-receptor (9) to a substrate (2) and a use of the peptide (1) in a detection device (15). Moreover, the invention relates to a polynucleotide encoding the peptide (1), an expression vector for expressing the peptide (1) and a peptide-linker conjugate.

## Description

The present invention relates to a peptide having an affinity for a formyl peptide receptor, referred to as a FP-receptor, a substrate comprising the peptide, a process for immobilizing the peptide to a substrate and a process for detecting the presence of a cell having a FP-receptor. The present invention further relates to a use of the peptide for immobilizing a cell having a FP-receptor to a substrate and a use of the peptide in a detection device. Moreover, the invention relates to a polynucleotide encoding the peptide, an expression vector for expressing the peptide and a peptide-linker conjugate.

Known methods in the field of the invention include the method disclosed in WO2016154620A1, wherein cellular activity is evaluated by placing a cell population on an area assaying for a dynamic behavior of the cell population as a function of time. The assayed dynamic behavior can include cellular activation, cellular inhibition, cellular interaction, protein expression, protein secretion, cellular proliferation, and other. The dynamic behavior and molecular profile is correlated for various purposes, such as screening cells and monitoring cellular activity.

However, this disclosure and other conventional approaches to receptor detection suffer from limited selectivity, non-specific interactions, and lack of integration into robust sensor platforms. Thus, there is a need for improved ligands, which are in particular capable of selective recognition of FP-receptors, preferably in a format amenable to immobilization on solid supports and incorporation into sensitive detection devices.

In view of the above, the invention is defined by the subject matter with the features of the independent claims.

A first aspect of the invention relates to a peptide having an affinity for a FP-receptor. The peptide consists of the amino acid sequence represented by any of SEQ ID NOS: 1 to 8 or any combination thereof. In addition, or in the alternative the peptide consists of a sequence represented by any of SEQ ID NOS: 1 to 8 or any combination thereof, in which at least one terminal amino acid is deleted, substituted or added and retains affinity for the FP-receptor. The peptide additionally or alternatively further consists of a sequence represented by any of SEQ ID NOS: 1 to 8 in which both terminal amino acids are deleted or substituted or added. Additionally, or alternatively, the peptide consists of a sequence in which one internal amino acid is deleted, substituted or added.

The amino acid sequence according to SEQ ID NO 1 is MVPIKI (Met-Val-Pro-Ile-Lys-Ile). The amino acid sequence according to SEQ ID NO 2 is MEQQNEK (Met-Glu-Gln-Gln-Asn-Glu-Lys). The amino acid sequence according to SEQ ID NO 3 is MKKFRK (Met-Lys-Lys-Phe-Arg-Lys). The amino acid sequence according to SEQ ID NO 4 is WKYMVm (Trp-Lys-Tyr-Met-Val-(D-Met)). The capital letter in the amino acid sequences represent L-amino acids in the one-letter codes. The small letters in the amino acid sequences represent D-amino acids in the one-letter code. For example, the small letter "m" in SEQ ID NO 4: WKYMVm represents D-methionine. The amino acid sequence according to SEQ ID NO 5 is f-MVPIKI ((formyl-Met)-Val-Pro-Ile-Lys-Ile). The amino acid sequence according to SEQ ID NO 6 is f-MEQQNEK ((formyl-Met)-Glu-Gln-Gln-Asn-Glu-Lys). The amino acid sequence according to SEQ ID NO 7 is f-MKKFRK ((formly-Met)-Lys-Lys-Phe-Arg-Lys). The amino acid sequence according to SEQ ID NO 8 is WKYMVm-NH₂ (Trp-Lys-Tyr-Met-Val-(D-Met-NH₂)).

Advantageously, these peptides offer specific binding to FP-receptors while allowing for sequence modifications that preserve receptor affinity. This enables optimization of stability, solubility or functionalization while maintaining target recognition.

A second aspect of the invention relates to a substrate for a detection device, in particular for a surface plasmon resonance spectrometer or a field-effect transistor. The substrate comprises a graphene monolayer. The graphene monolayer is arranged on a surface, in particular a dielectric surface. The peptide according to the first aspect is immobilized on the graphene monolayer and/or an antibody is immobilized on the graphene monolayer. The antibody in particular has an affinity for a virus. Advantageously, the graphene monolayer significantly enhances detection efficiency.

The substrate is preferably a graphene substrate, a graphite substrate, or a graphene covered substrate, in particular with a graphene monolayer, or a glass substrate or a gold substrate or a polymer substrate. The substrate is preferably an anchor point, to which the peptide and/or the antibody are immobilized onto. The substrate may be a substantially flat surface. The immobilization may be direct or indirect. The indirect immobilization may be achieved via a linker between the peptide and the substrate and/or between the antibody and the substrate. The immobilization onto the substrate may occur via covalent bonding, π-π-stacking, Van-der-Waals interactions and/or hydrogen bonding. The graphene layer is preferably a graphene monolayer. Advantageously, immobilizing antibodies allows for specific detection of targets such as viruses or other pathogens. The graphene monolayer significantly enhances detection efficiency. The substrate beneficially leads to a reproducible signal enhancement of up to 600% in comparison to non-covered gold substrates. Moreover, the substrate allows for discrimination between different cell types and virus types, which is currently not possible with conventional substrates. Advantageously, such immobilization enables robust surface functionalization suitable for biosensor applications. Graphene and gold provide conductive and stable platforms that support both optical and electrical detection strategies.

A third aspect of the invention relates to a detection device. The device may comprise the substrate according to the second aspect. The substrate may comprise at least one detection zone. The detection zone has preferably a detection surface formed on a conductive layer capable of supporting surface plasmon resonance, SPR. The device may further comprise an illumination system configured to incident radiation onto the detection surface to excite surface plasmons. The device preferably comprises a detection system configured to detect changes in the reflected and/or transmitted radiation resulting from interactions at the detection surface. The detection surface is in particular formed at least partly by the substrate and especially by the graphene monolayer. Preferably, the device comprises a fluidic system which is configured to deliver a sample to the detection surface. Advantageously, the detection device allows label-free monitoring of receptor-ligand interactions through SPR. This facilitates real-time kinetic studies or quantification of target analytes with high sensitivity.

A fourth aspect of the invention relates to a detection device. The device may comprise the substrate according to the second aspect. The substrate may comprise at least one detection zone supporting the graphene monolayer. The device preferably includes a field-effect transistor, FET, structure comprising a source electrode, a drain electrode, and a gate electrode. The graphene monolayer may extend between the source electrode and the drain electrode, forming a conductive channel. The gate electrode is preferably configured to modulate a conductivity of the conductive channel. The device may comprise a detection system configured to detect changes in the electrical properties of the conductive channel. Preferably, the device comprises a fluidic system configured to deliver a sample to the detection surface. The detection zone and the detection surface may be identical. The detection device is preferably configured for measuring changes in conductivity, resistance, or other electrical properties resulting from the interaction between the sample and the substrate. The detection device is preferably configured for detecting the presence and/or concentration of target analytes based on changes in the surface plasmon resonance conditions at the detection zone. Advantageously, the use of graphene-based FETs enables highly sensitive electronic biosensing. Interaction with the peptide at the graphene surface modulates the electrical signal, providing a rapid, miniaturized and label-free detection modality.

A fifth aspect of the invention relates to a process for immobilizing the peptide according to the first aspect. The process comprises steps of providing a substrate, the peptide, and a linker having an affinity for the substrate. In a first option, the linker is contacted with the substrate to immobilize the linker on the substrate. The peptide is then connected to the linker to obtain a peptide-linker conjugate and immobilize the peptide on the substrate. In a second option, the peptide is first connected to the linker to obtain a peptide-linker conjugate. The peptide-linker conjugate is then contacted with the substrate for immobilizing the peptide on the substrate. Advantageously, the process allows flexible immobilization strategies compatible with various substrates and application-specific linker chemistries. Pre- or post-functionalization pathways enhance process adaptability.

A sixth aspect of the invention relates to a process for detecting the presence of a cell having a FP-receptor and/or a virus having an affinity for the antibody. This process preferably comprises steps of providing a substrate equipped with the peptide according to the first aspect and/or an antibody. The process further comprises providing a sample comprising a plurality of cells, of which at least one cell has a FP-receptor and/or viruses of which at least one virus has an affinity for the antibody. A reference property is measured in the absence of the sample. The sample is then contacted with the substrate. A contact property is measured during and/or after the contacting of the substrate with the sample.

The reference property is compared to the contact property to determine the presence or absence of the at least one cell having a FP-receptor and/or the least one virus having an affinity for the antibody in the sample. Preferably, the steps of the process or the processes take place consequently in the order mentioned in the claims or the description. Advantageously, this detection method allows selective identification of target cells based on receptor expression. Quantitative or qualitative assessment is enabled through physical property measurement.

A seventh aspect of the invention provides the use of the peptide according to the first aspect. The use is for immobilizing a cell having a FP-receptor to a substrate. Advantageously, such use enables cell capture or patterning applications. It facilitates the development of biosensors, tissue models, or diagnostic devices involving receptor-expressing cells.

An eighth aspect of the invention provides the use of the peptide according to the first aspect. The use is for determining the presence or absence of at least one cell having a FP-receptor in a sample, in particular in a detection device. Advantageously, the integration of receptor-specific peptides in detection devices supports high specificity in cell detection assays.

A ninth aspect of the invention provides a polynucleotide encoding the peptide of the first aspect. Advantageously, synthetic or recombinant production of the peptide is enabled, supporting scalable and reproducible manufacture.

A tenth aspect of the invention provides an expression vector for expressing the peptide according to the first aspect. Advantageously, the expression vector allows efficient intracellular or extracellular production in host systems.

A preferred aspect provides a transformant obtained by transforming a host cell by introducing the expression vector for expressing the peptide of the first aspect and/or a peptide fusion protein comprising the peptide of the first aspect and a further protein. In particular the expression vector comprises the polynucleotide of the ninth aspect and/or a further polynucleotide encoding the further protein. The further polynucleotide is in particular linked to the polynucleotide of the tenth aspect. Advantageously, the transformant serves as a production system for functional peptides or peptide fusion constructs. It supports both peptide characterization and practical applications.

A preferred aspect provides a peptide fusion protein comprising the peptide of the first aspect and a further protein. The peptide fusion protein is preferably obtainable from the transformant according to the previous preferred aspect. Advantageously, such fusion proteins allow dual-function constructs or facilitate purification, stabilization or targeting.

An eleventh aspect of the invention provides a peptide-linker conjugate comprising the peptide of the first aspect and a linker. The linker preferably has an affinity for a graphene substrate or a gold substrate. The peptide is in particular connected to the linker. Advantageously, peptide-linker conjugates simplify immobilization and ensure orientation-controlled binding to sensor surfaces.

Preferred embodiments of the invention are defined in the dependent claims. In the context of the present invention, it shall be understood that the peptides, substrates, devices, processes or uses or other subject-matter of the claims and/or the description have similar and/or identical preferred embodiments with each other, in particular as defined in the dependent claims and as disclosed herein.

The present invention is in particular based on the idea of using FP-receptors for attachment and detection of cells on a substrate. Surprisingly it has been observed, that FP-receptors are a very suitable target as they allow for an extraordinarily highly selectivity. Moreover, the binding of the peptides to the FP-receptors is advantageously robust, allowing for efficient processes. FP-receptors are key components in immune cell signaling. Furthermore, FP-receptor are crucial for regulating inflammation, host defense mechanisms, and tissue repair.

The invention is further based on the idea of providing a highly sensitive, detection device that uses the synthetic peptides to selectively recognize FP-receptors on cell surfaces, in particular on immune cell surfaces, which is highly advantageous for the early identification of immune responses and inflammation related conditions. Surprisingly it has been found that graphene-based detection devices, in particular with a graphene monolayer substrate, that operate on both surface plasmon resonance and graphene field-effect transistor platforms show a significant efficiency enhancement. The detection devices preferably employ the synthetic peptides for the specific identification and real-time monitoring of FP-receptors, in particular on the membranes of immune cells. Utilizing a system for cell surface receptors such as FP-receptors is highly beneficial for mediating a range of physiological and pathological cellular responses. The current invention allows for accurate detection and selective binding of such receptors, as this is beneficial in biomedical diagnostics, drug screening, and cellular profiling.

The invention is further based on the idea of providing a process for determining two parameters in a single measurement, namely (a) the presence of a cell with a FP-receptor and (b) the amount of detected FP-receptors. Advantageously, the detection results are obtained fast, preferably in seconds to less than one minute. This preferably allows monitoring of activity parameters of living cells with a high time resolution, in particular in real time at low costs.

According to a preferred embodiment, at least one amino acid of the amino acid sequence is connected to a fluorophore. Preferably, the amino acid connected to the fluorophore is lysine. More preferably, the fluorophore is connected to the lysine which is the one closest to the carboxyl terminus, in particular, if the amino acid sequence includes more than one lysine.

According to a preferred embodiment, the fluorophore is connected to the lysine underlined in the amino acid sequence according to SEQ ID NO 1: MVPIKI. According to a preferred embodiment, the fluorophore is connected to the lysine underlined in the amino acid sequence according to SEQ ID NO 2: MEQQNEK. According to a preferred embodiment, the fluorophore is connected to the lysine underlined in the amino acid sequence according to SEQ ID NO 3: MKKFRK. According to a preferred embodiment, the fluorophore is connected to the lysine underlined in the amino acid sequence according to SEQ ID NO 4: WKYMVm. In other preferred embodiments, the fluorophore is connected to the corresponding lysine of any one of SEQ ID NOs 5 to 8. Advantageously, fluorophore labelling enables visualization, quantification and tracking of receptor binding events. Labelling at the C-terminal lysine provides predictable conjugation sites while minimizing functional disruption.

According to a preferred embodiment the fluorophore is selected from the list comprising or consisting of: fluorescein-based dyes such as in particular fluorescein isothiocyanate (FITC), rhodamine derivatives such as in particular tetramethylrhodamine isothiocyanate (TRITC), cyanine dyes such as in particular Cy3 and Cy5, Alexa Fluor^{™} dyes such as in particular Alexa Fluor^{™} 488, and boron-dipyrromethene (BODIPY) derivatives or any combination thereof. Each of these fluorophores is characterized by a defined excitation and emission spectrum, making them suitable for optical detection using standard fluorescence instrumentation. In particular fluorescein (FITC) is a widely used green-emitting fluorophore having an excitation wavelength around 495 nm and an emission wavelength around 519 nm. Advantageously, it may be readily coupled to peptides via isothiocyanate chemistry targeting primary amine groups. Rhodamine-based fluorophores, such as for example TRITC, exhibit excitation and emission maxima around 550 nm and 570 nm, respectively, and advantageously provide improved photostability compared to fluorescein. Cyanine dyes, such as Cy3 and Cy5, beneficially enable detection in the red and near-infrared spectral ranges, typically at 550 nm/570 nm and 649 nm/670 nm (excitation/emission), respectively. These dyes are particularly well-suited for multiplex detection applications or for use in Förster Resonance Energy Transfer (FRET) assays. Alexa Fluor dyes advantageously offer improved quantum yield and photostability across a broad range of spectral variants. For instance, Alexa Fluor^{™} 488 has excitation/emission properties similar to fluorescein but with superior resistance to photobleaching and pH insensitivity. Advantageously, BODIPY-based dyes provide narrow emission spectra and high fluorescence intensity with reduced sensitivity to environmental quenching. They are particularly advantageous for labeling the peptides due to their compact size and high stability. Advantageously, the use of fluorophores covalently linked to the peptide facilitates sensitive and specific detection of peptide-target interactions by means of fluorescence-based techniques and spatial localization of fluorescent signals on an immobilized substrate allows detection of discrete binding zones, which is beneficial in microstructured detection devices and sensor arrays. Advantageously, the integration of fluorophore-conjugated peptides in surface plasmon resonance (SPR) or field-effect transistor (FET)-based biosensors provides a dual-mode readout, increasing the robustness of detection and reducing false-positive signals.

According to a preferred embodiment, the at least one terminal amino acid substituted is methionine. Preferably, the terminal substituted methionine is N-formylmethionine. According to a preferred embodiment, the terminal methionine with the prefix "f" is N-formylmethionine according to SEQ ID NO 1: f-MVPIKI, which corresponds to SEQ ID NO 5. According to a preferred embodiment, the terminal methionine with the prefix "f" is N-formylmethionine according to SEQ ID NO 2: f-MEQQNEK, which corresponds to SEQ ID NO 6. According to a preferred embodiment, the terminal methionine with the prefix "f" of is N-formylmethionine according to SEQ ID NO 3: f-MKKFRK, which corresponds to SEQ ID NO 7. According to a preferred embodiment, the terminal amino acid substituted is D-methionine. Preferably, the terminal substituted amino acid is D-methioninamide. Preferably, the terminal substituted methionine is methioninamide. According to a preferred embodiment, the terminal D-methionine with the suffix "NH₂" is D-methioninamide according to SEQ ID NO 4 is WKYMVm-NH₂ (Trp-Lys-Tyr-Met-Val-(D-Met-NH₂)), which corresponds to SEQ ID NO 8.

According to a preferred embodiment, the peptide has a hydrophobicity according to a GRAVY score from -3.0 to 2.0, preferably from -2.8 to 1.6. Most preferably the hydrophobicity equals -2.8 or -1.9 or 0.3 or 1.6. According to a preferred embodiment, the peptide has a charge, in particular a residual charge. Preferably the charge is from -2 to +4. Advantageously, these peptide properties allow for a high selectivity towards FP-receptors and allow for a beneficially sensitive detection of cells with an FP-receptor in the detection devices.

According to a preferred embodiment, the peptide consisting of the amino acid sequence represented by SEQ ID NO 5, f-MVPIKI, has a higher affinity to the FP-receptor than the peptide consisting of the amino acid sequence represented SEQ ID NO 6, f-MEQQNEK. Optionally the peptides consisting of the amino acid sequence represented by SEQ ID NO 5, 7 and 8 have a higher affinity to the FP-receptor than the peptide consisting of the amino acid sequence represented SEQ ID NO 6, f-MEQQNEK. In other words, the peptide consisting of the amino acid sequence represented SEQ ID NO 6, f-MEQQNEK optionally has the lowest affinity to FP-receptor, if compared to the other peptides. Advantageously, this allows for designing substrates with areas of higher and lower affinities for the FP-receptor, which is beneficial for particular characterization protocols.

According to a preferred embodiment, the peptide has an affinity for a FP-receptor 1 and/or an affinity for a FP-receptor 2 an/or affinity for a FP-receptor 3. In a preferred embodiment, the peptide has a higher affinity for the FP-receptor 1 than for the FP-receptor 2. In an alternative embodiment, the peptide has a higher affinity for the FP-receptor 2 than for the FP-receptor 1. Preferably, the peptide consisting of the amino acid sequence of SEQ ID NO 5, f-MVPIKI, has a specific affinity for the FP-receptor 1. More preferably, the peptide consisting of the amino acid sequence of SEQ ID NO 5, f-MVPIKI, has a higher affinity for the FP-receptor 1 than for the FP-receptor 2. Preferably, the peptide consisting of the amino acid sequence of SEQ ID NO 7, f-MKKFRK, and/or the peptide consisting of the amino acid sequence of SEQ ID NO 4, WKYMVm, has a specific affinity for the FP-receptor 2, optionally only for FP-receptor 2 and not for FP-receptor 1. Preferably, the peptide consisting of the amino acid sequence of SEQ ID NO 8, WKYMVm-NH₂, has a specific affinity for the FP-receptor 2. More preferably, the peptide consisting of the amino acid sequence of SEQ ID NO 8, WKYMVm-NH₂, has a higher affinity for the FP-receptor 2 than for the FP-receptor 1.

According to a preferred embodiment, the substrate comprises two or more different peptides having an affinity for a FP-receptor. In particular the substrate comprises two or more different peptides or all of the peptides consisting of the amino acid sequence represented by any of the SEQ ID NOS: 1 to 8.

According to a preferred embodiment, the substrate comprises the peptide consisting of the amino acid sequence of SEQ ID NO 5, f-MVPIKI, which has a specific affinity for the FP-receptor 1 and/or peptide consisting of the amino acid sequence of SEQ ID NO 7, f-MKKFRK, which has a specific affinity for the FP-receptor 2.

According to a preferred embodiment, the substrate is a graphene monolayer. Advantageously, a single layer of graphene can significantly enhance the FP-receptor detection performance, for example due to distinctive optical properties, minimal thickness, and large surface-to-volume ratio. The substrate may be transparent. The substrate may also be a glass slide covered with the graphene monolayer or a gold substrate covered with the graphene monolayer or a glass slide covered with a gold layer, which gold layer is at least partly covered with the graphene monolayer.

According to a preferred embodiment, the substrate is configured for an optical sensor and/or an electrochemical sensor, in particular for a surface plasmon resonance spectrometer. The substrate may preferably comprise a glass substrate, which is coated with a conductive layer, more in particular with a gold layer, a silver layer, a chromium layer, and/or a platinum layer. The conductive layer is preferably coated with the graphene monolayer and/or another signal enhancing nanomaterial.

According to a preferred embodiment, the substrate is configured for a field-effect transistor. The substrate may comprise: a glass substrate and/or a polymer substrate, which is or which are coated with the graphene monolayer and/or another semiconducting material used as a channel of the field-effect transistor.

According to a preferred embodiment, the substrate comprises a cell. The cell has in particular at least one FP-receptor. The cell is preferably immobilized on the substrate via the peptide having an affinity to the cells' FP-receptor. Advantageously, such substrates enable spatially controlled cell capture, supporting selective cell detection, analysis or enrichment.

According to a preferred embodiment, the substrate comprises a virus. The virus has in particular an affinity to the antibody. The virus is preferably immobilized on the substrate via the antibody. Advantageously, such substrates enable spatially controlled virus capture, supporting selective virus detection, analysis or enrichment.

According to a preferred embodiment, the detection device for surface plasmon resonance is combined with the detection device comprising the field-effect transistor structure. In particular, this embodiment comprises
the substrate with the at least one detection zone with the detection surface formed on the conductive layer capable of supporting surface plasmon resonance, wherein the detection zone supports the graphene monolayer;
the illumination system configured to incident radiation onto the detection surface to excite surface plasmons,
the detection system configured to detect changes in the reflected and/or transmitted radiation resulting from interactions at the detection surface,
the field-effect transistor structure comprising the source electrode, the drain electrode and the gate electrode,
wherein the graphene monolayer extends between the source electrode and the drain electrode, forming the conductive channel and wherein the gate electrode is configured to modulate the conductivity of the conductive channel,
the detection system, which is configured to detect changes in the electrical properties of the conductive channel, and
preferably the fluidic system, which is configured to deliver the sample to the detection zone.

According to a preferred embodiment the process for immobilizing the peptide comprises the step of: (a3) capping any remaining linkers. This step is preferably performed after step (a2) connecting the peptide to the linker for obtaining the peptide-linker conjugate and immobilizing the peptide on the substrate. According to a preferred embodiment the substrate comprises capped linkers, more preferably ethanolamine capped linkers. Advantageously, capping can enhance the robustness and sensitivity of the detection devices.

According to a preferred embodiment the fluidic system is a flow cell configured to deliver the sample. The flow cell may comprise a plurality of channels for delivering the sample to spatially different parts of the substrate, which may be equipped with different peptides and/or antibodies.

These and other aspects of the invention will be apparent from and elucidated with reference to the embodiment(s) described hereinafter. In the following drawings
- Figure 1: shows a schematic illustration of a process for immobilizing a peptide to a substrate;
- Figure 2: shows a schematic flow cell for contacting a sample with a substrate;
- Figure 3: shows a schematic illustration of a detection device for detecting surface plasmon resonance;
- Figure 4: shows a schematic illustration of a detection device with a field-effect transistor;
- Figures 5 and 6: show schematic illustrations of diagrams representing data measured with the detection device for detecting surface plasmon resonance of figure 3;
- Figures 7 and 8: show schematic illustrations diagrams representing data measured with the detection device with a field-effect transistor of figure 4;
- Figures 9 to 12: show schematic illustrations diagrams representing data measured with the detection device for detecting surface plasmon resonance of figure 3;
- Figures 13 to 18: show schematic illustrations of diagrams representing data measured with the detection device a field-effect transistor of figure 4;
- Figure 19: shows a schematic illustration of the peptides;
- Figures 20a to 20f: show the immobilization of different peptides and related data;
- Figures 21a to 21f: show peptide and cell interactions and related data;
- Figures 22a to 22e: show the immobilization of antibodies and related data.
- Figures 23a to 23f: show antibody and virus interactions and related data.

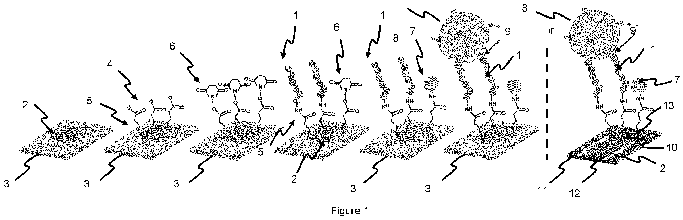

Figure 1 shows a schematic illustration of a process for immobilizing a peptide 1 to a substrate 2. From left to right, Figure 1 shows a gold surface 3 plasmon resonance slide, which is coated with graphene as the substrate 2. Therefore, the graphene substrate 2 is typically grown on a copper substrate, covered with a polymer, released from the copper substrate and transferred onto the gold covered slide 3, for example a gold coated glass slide. After removing of the polymer covering, the gold coated slide 3 is covered with a graphene substrate 2, ideally a graphene monolayer substrate 2.

In the second illustration from the left, a carboxylic acid 4 functionalized pyrene linker 5, for example a butyric acid functionalized pyrene is attached to the graphene substrate 2 via π-π-stacking, i.e. Van-der-Waal interactions. As a consequence, the carboxylic acid 4 functionalized pyrene linker 5 is not covalently bound to the graphene substrate 2 but instead attached substantially strong enough thereto via the π-π-stacking. Due to repulsive forces, the carboxylic acid functional group 4 is oriented away from the graphene substrate 2.

According to the third illustration from the left, the carboxylic acid functional group 4 is subsequently converted into an activated succinimide 6. This activation can be achieved by standard reagents and succinimidation protocols, for example by first adding N-hydroxysuccinimide, a small amount of an organic base in an anhydrous solvent and then adding a coupling reagent such as dicyclohexylcarbodiimide or 1-ethyl-3-(3-dimethylaminopropyl)carbodiimide to form a highly reactive activated acid intermediate. N-hydroxysuccinimide then reacts to from a more stable activated carboxylic acid derivative.

According to the fourth illustration from the left, the activated succinimide 6 is coupled with the peptide 1 for immobilizing the peptide 1 on the substrate 2.

The peptide 2 is covalently bound via an amide bond to the linker 5 in a grafting-to approach. In particular, a lysine of the peptide 1 sequence may be coupled to the linker 5.

According to the fifth illustration from the left, any remaining and unreacted activated succinimide 6 are capped 7 with a capping reagent. The capping reagent is preferably ethanol amine. Interestingly, the capping 7 can beneficially influence the surface plasmon resonance and enhance sensitivity.

According to the sixth illustration from the left, a cell 8 with a FP-receptor 9 is interacting with the peptide 9. FP-receptor-mediated interactions between cells and the peptides are conducted after immobilizing the peptides 1 on the substrate 2 via amine coupling chemistry. When cells 8 are introduced as analytes by flowing them over the substrate 2, the successful attachment of the cells 8 via their FP-receptors 9 to the substrate 2 is observed by surface plasmon resonance. In particular, the cell 8 is immobilized onto the substrate 2 via the peptide 1 and the linker 5.

The illustration on the right shows a system for a field-effect transistor system. The substrate 2 is a polymer substrate. A graphene layer is in a detection zone 10 of the substrate 2. The peptide is immobilized on the graphene layer 2 in a similar manner as shown in the illustration on the left side and explained previously. The field-effect transistor structure comprises a source electrode 11, a drain electrode 12 and a gate electrode 13. The source electrode 11, drain electrode 12 and gate electrode 13 are wires embedded in the polymer substrate 2, preferably gold wires or platinum wires. The polymer substrate 2 between the gold wires acts as an isolator. The graphene layer in the detection zone 10 extends between the source electrode 11 and the drain electrode 12, forming a conductive channel. The gate electrode 13 is configured to modulate a conductivity of the conductive channel. The graphene layer advantageously facilitates the detection of a cell 8 with FP-receptors 9 interacting with the peptide 1.

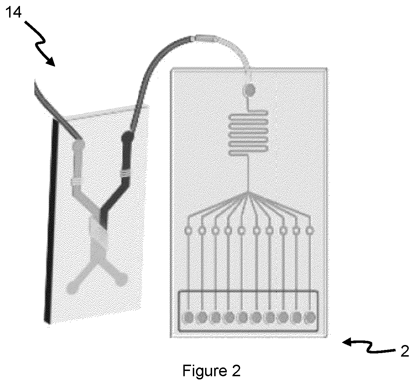

Figure 2 shows a schematic flow cell for contacting a sample of cells 8 with a substrate 2. The flow cell may be employed as a fluidic system 14 for delivering the sample. In particular, the fluidic system 14 allows for separating erythrocyte cells from leukocyte cells. Subsequently, the leukocyte cells are contacted as sample cells 8 with the substrate 2 and the peptides 1 attached thereto (see Figure 1).

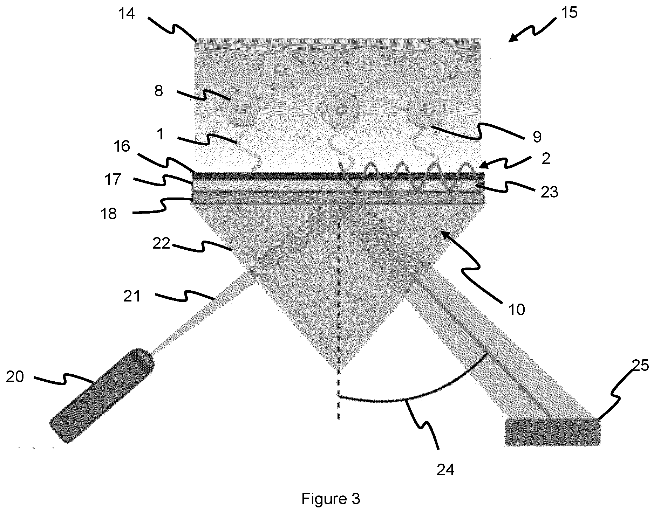

Figure 3 shows a schematic illustration of a detection device 15 for detecting surface plasmon resonance. The detection device 15 comprises a substrate 2. The substrate 2 includes a graphene monolayer 16, a gold layer 17, and a glass slide 18. The gold layer 17 is positioned between the graphene layer 16 and the glass slide 18. In other words, the glass slide 18 is coated with the gold layer 17, which in turn is coated with the graphene layer 16. The peptide 1 is immobilized on the graphene layer 16, as described previously. A cell 8 is interacting with the peptide 1 via the cells' FP-receptor 9. This multilayer structure substrate 2 enables the functional integration of optical and chemical components relevant for surface plasmon resonance (SPR) detection. A detection zone 10 is formed on the substrate 2 and incorporates the graphene layer 16 as part of its detection surface. The detection zone 10 is configured to interact with a sample with cells 8 introduced by the fluidic system 14, such as a flow cell. The peptide 1 is immobilized in the detection zone 10 and is exposed to cells 8 delivered by the fluidic system 14. The detection device 15 further comprises a light source 20 configured to emit a light beam 21. The light beam 21 is directed through a prism 22 toward the detection zone 10 with the detection surface at an angle suitable for exciting surface plasmons. Upon excitation of surface plasmons, a plasmon wave 23 is resulting at the interface of the graphene layer 16 and the gold layer 17. The reflected portion of the light beam 21 undergoes a change in intensity depending on the presence and interaction of biological analytes at the detection surface. This change is observed at a specific resonance angle 24. A detector 25 is positioned to capture the reflected beam and determine the resonance angle 24, thereby enabling sensitive detection of interaction events at the peptides 1 in the detection zone 10. The amount of reflected light correlates with the number of binding events and thereby with the number of cells and the amount of FP-receptors interacting with the peptides 1. Advantageously this allows for quantitative analysis. The substrate 2 may also be transparent to the light beam. In such cases a transmission measurement is possible.

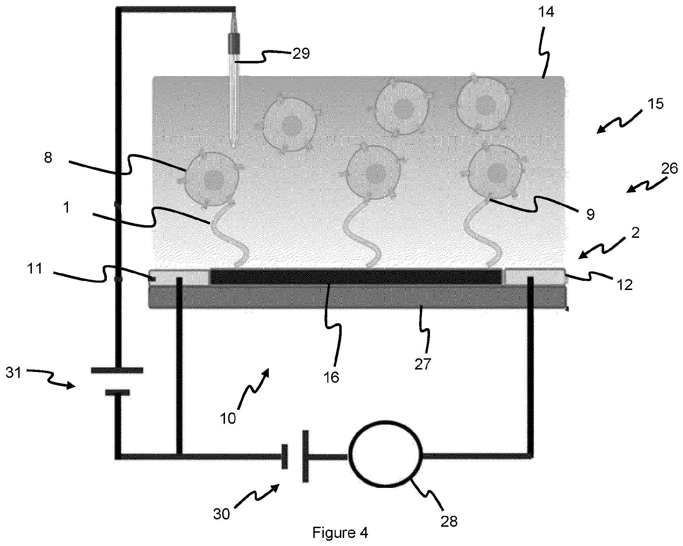

Figure 4 shows a schematic illustration of a detection device 15 with a field-effect transistor structure 26. The detection device 15 comprises a substrate 2 with a detection zone 10. The detection zone 10 includes a graphene monolayer 16, which is grafted onto a plastic material 27 as the substrate 2. The graphene layer 16 extends between a source electrode 11 and a drain electrode 12, thereby forming a conductive channel. The source electrode 11, the drain electrode 12, and a gate electrode 13 (see Figure 1) are gold wires embedded in the plastic material 27. These three electrodes collectively form the field-effect transistor structure 26. A fluidic system 14 is arranged in connection with the detection device 15. The fluidic system 14 is configured as a flow cell, which is filled with a liquid sample containing cells 8. The peptides 1 are immobilized on the surface of the graphene layer 16. The immobilized peptides 1 are capable of interacting with FP-receptors 9 of the cells 8 in the sample. Modulation of the electrical properties of the conductive channel enables detection of changes in conductivity or resistance caused by interactions between the sample cells 8 FP-receptors 9 and the immobilized peptides 1. A reference electrode 29, in particular a Ag/AgCl reference electrode is arranged within the flow cell and is in contact with the sample solution containing the cells 8. A voltage source V_{DS} 30 and an amperemeter 28 are electrically connected in series with the source electrode 11 and the drain electrode 12. The amperemeter 28 is configured to measure the drain-source current I_{DS} flowing through the conductive channel between the source electrode 11 and the drain electrode 12. A second voltage source V_{GS} 31 is electrically connected between the Ag/AgCl reference electrode 29 and the source electrode 11.

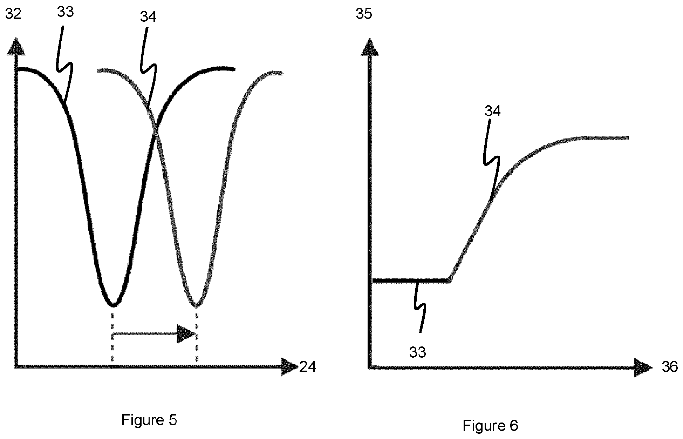

Figures 5 and 6 show schematic illustrations of diagrams representing data measured with the detection device for detecting surface plasmon resonance of figure 3. In Figure 5 the ordinate represents the measured intensity, and the abscissa represents the resonance angle 24. The curve on the left side represents the data measured in the absence of cells 8, in other words a reference property 33. The curve on the left side represents the data measured in the presence of a sample containing cells, in other words a contact property 34. As can be seen in Figure 5, the curve of the contact property 34 is shifted towards larger resonance angles 24 in comparison to the reference property 33. Especially the minimum of the contact property 34 curve is shifted to the right when compared to the minimum of the reference property 33 curve, as indicated by the arrow in Figure 5. This confirms the interaction of FP-receptors 9 with the peptides 1 (see Figure 3).

In Figure 6 the ordinate represents the surface plasmon-polariton resonance (SPPR) signal 35 in refractive index units (RU). The abscissa represents the time 36. The curve in Figure 6 is initially constant, while the reference property 33 is measured. After addition of the sample containing cells 8 with FP-receptors 9 (see Figure 3) the curve kinks and then increases to higher SPPR values while measuring the contact property 34, thereby confirming the interaction of FP-receptors 9 with the peptides 1.

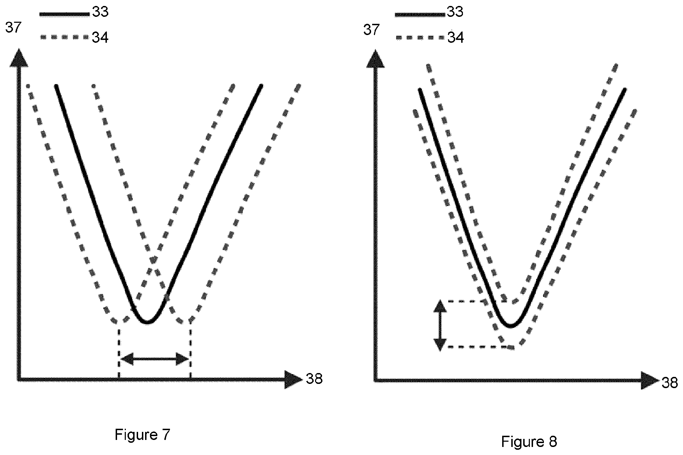

Figures 7 and 8 show schematic illustrations of diagrams representing data measured with the detection device 15 with a field-effect transistor of Figure 4. In Figures 7 and 8 the ordinate represent the current I_{DS} measured via the amperemeter 28 and the abscissa represent the voltage V_{GS} applied by the voltage source 30 (see Figure 4). The curves in dashed lines represent the data measured as contact property 34 in the presence of cells 8 with FP-receptors 9. The curve in solid line represents the reference property 33 measured in the absence of cells 8 with FP-receptors 9. As can be seen from Figures 7 and 8, the contact property 34 curves are shifted in comparison to the reference property 33 curves, for example to the left and right, as displayed in Figure 7, towards lower or higher voltages respectively. In Figure 8 the contact property 34 curves are shifted in comparison to the reference property 33 curves upwards or downwards, towards higher or lower currents, respectively. The curves shift can be easily observed by regarding the minima of the curves, as indicated by the arrows in Figures 7 and 8.

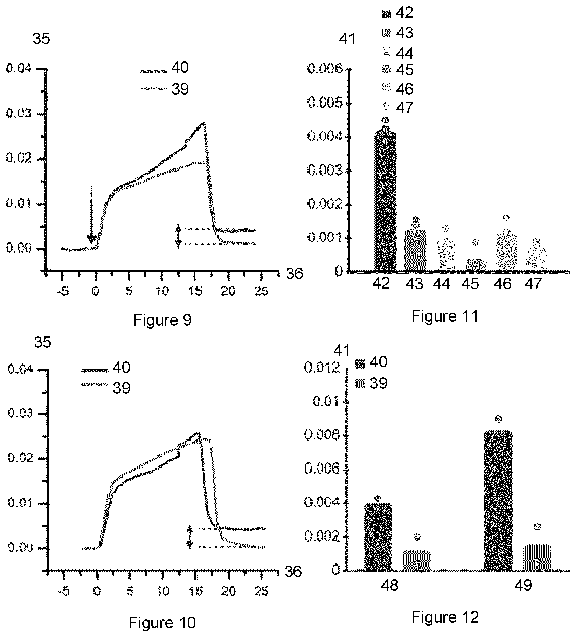

Figures 9 to 12 show schematic illustrations diagrams representing data measured with the detection device for detecting surface plasmon resonance of Figure 3. In Figures 9 and 10 the ordinates represent the surface plasmon-polariton resonance (SPPR) signal 35 in refractive index units (RU). The abscissa represents the time 36. The two curves in Figure 6 are initially constant at around SPPR 0.00 while measuring the reference property 33. After addition of a sample containing cells 8, at time 0, indicated by the vertical arrow in Figure 9, both curves increase first and decrease later. The curve returning to the initial level of SPRR 0.00 is the curve measure with a control sample 39. The other curve remains at a SPRR value higher than 0, approximately at 0.05, as indicated by the arrow and the dashed horizontal lines in Figures 9 and 10. This curve is measured with a target sample 40 containing cells 8 with FP-receptors 9 (see Figure 3). In Figure 9 the target sample 40 and the control sample 39 contained HEK R⁺ cells. In Figure 10 the target sample 40 contained neutrophiles and the and the control sample 39 contained blocked neutrophiles. The neutrophiles may be immune cells such as leukocytes. The shift of the remaining SPRR value between the target sample 40 and the control sample 39 at higher times clearly indicates successful immobilization of the cells 8 or neutrophiles contained in the target sample 40 on substrates 2 via the peptides 1.

In Figures 11 and 12 the ordinate represents a mean surface plasmon-polariton resonance signal 41 in refractive index units (RU). In the bar chart of Figure 11 the bars from left to right represent data from: target HEK R⁺ cells 42, control HEK R⁺ cells 43, target HEK R⁻ cells 44, control HEK R⁻ cells 45, blocked target HEK R⁺ cells 46 and blocked control HEK R⁺ cells 47. As can be seen from Figure 11, the target HEK R⁺ cells 42 clearly show the strongest mean surface plasmon-polariton resonance signal 41 if compared to the other cells. In the bar chart of Figure 12 the bars from left to right represent data from: a target sample 40 with neutrophiles of a first donor 48, a control sample 39 with blocked neutrophiles of the first donor 48. Then on the right a target sample 40 with neutrophiles of a second donor 49 and a control sample 39 with blocked neutrophiles of the second donor 49. For the target sample 29 neutrophile of the first donor 48 and the second donor 49 a stronger mean surface plasmon-polariton resonance signal 41 can be clearly observed in Figure 12 if compared to the control sample 40 neutrophiles of the first donor 48 and the second donor 49.

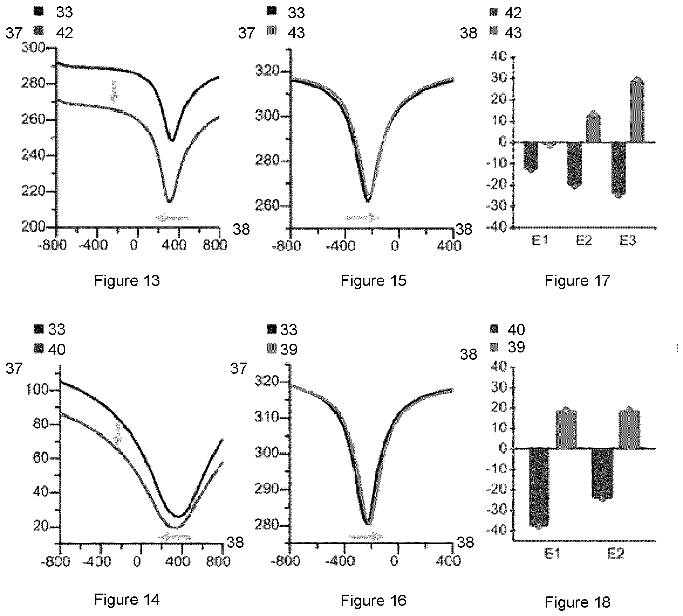

Figures 13 to 18 show schematic illustrations of diagrams representing data measured with the detection device 15 with a field-effect transistor of Figure 4. The ordinate in Figures 13 to 16 represents a drain current 37 in µA. The abscissa represents a gate voltage 38 in mV. In Figure 13 the curve of the data of a sample with target HEK R⁺ cells 42 is significantly shifted if compared to the reference property 33 curve. As indicated by the arrows in Figure 13 the minimum of the curve of target HEK R⁺ cells 42 is shifted downwards to a lower drain current 37 value and to the left to a lower gate voltage 38 value. According to Figure 15, this behavior cannot be observed when a sample with control HEK R⁺ cells 43 is used instead. As indicated by the arrow in Figure 15, the minimum of the curve control HEK R+ cells 43 is shifted to higher gate voltage 38 values in comparison to the reference property 33 curve. In Figure 14 the curve of the data of a target sample 40 with neutrophiles is significantly shifted if compared to the reference property 33 curve. In Figure 16 the curve of the data of a control sample 39 with blocked neutrophiles is not shifted as significantly, if compared to the reference property 33 curve and to the shift observed in Figure 14.

The ordinate in Figures 17 and 18 represents a dirac voltage 38 V_{Dirac} in mV. The bar charts represent data for different replication experiments. In Figure 17 the results of the evaluation of Figure 13 and Figure 14 is displayed for three different replication experiments E1 to E3. The experimental data for all experiments E1 to E3 show a negative dirac voltage 38 for the target HEK R⁺ cells 42 and a positive or only a small negative dirac voltage 38 for the control HEK R⁺ cells 43. A similar behavior is observed in Figure 18 for the results of the evaluation of Figures 14 and 16 for two different replication experiments E1 and E2. The experimental data for all experiments E1 to E2 show a negative dirac voltage 38 for the target sample 40 with neutrophiles and a positive dirac voltage 38 for the control sample 39 with blocked neutrophiles. By evaluation of these significant differences, the presence of FP-receptors 9 in a sample can be clearly determined and distinguished from a sample without FP-receptors.

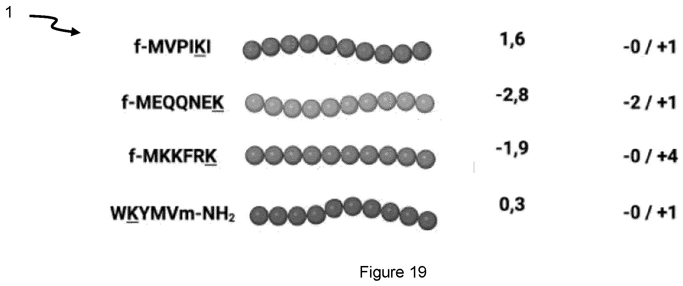

Figure 19 shows a schematic illustration of the peptides 1. Next to the peptide sequence from the left it is displayed the hydrophobicity according to a GRAVY score and next the charge of residues. A negative hydrophobicity value qualifies the peptide as hydrophilic, while a negative hydrophobicity value qualifies the peptide as hydrophobic. The peptide with the amino acid sequence f-MVPIKI has a hydrophobicity of 1.6 and a charge of +1. The peptide with the amino acid sequence f-MEQQNEK has a hydrophobicity of -2.8 and a charge of +1. The peptide with the amino acid sequence f-MKKFRK has a hydrophobicity of -1.9 and a charge of +4. The peptide with the amino acid sequence WKYMVm-NH2 has a hydrophobicity of 0.3 and a charge of +1.

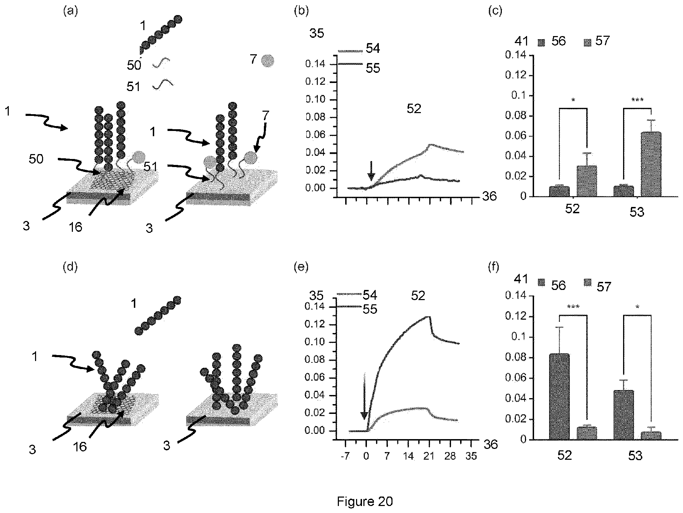

Figure 20 shows the immobilization of differently charged peptides 1 on gold and graphene substrates 2. In Figure 20a a schematic illustration of peptide 1 immobilization via amine coupling chemistry on plain gold and graphene-coated SPR sensor chips is shown. In Figure 20b SPR signal from the interaction of f-MVPIKI 52 with linker 50, 51 molecules coated on bare gold 55 and graphene-coated 54 SPR substrate 2 sensor chips is shown. In Figure 20c, a bar graph of mean SPR signals 41 for peptide 1 interaction with linker 50, 51 molecules on plain gold (right side in blue) and graphene-coated SPR sensor chips 57 (left side in red) with linkers 50, 51 is shown. Figure 20d schematically illustrates physical adsorption of peptide 1 on plain gold (right) and graphene-coated (left) SPR sensor chips without linkers 50, 51. Figure 20e shows SPR signal 35 from the physical adsorption of F-MVPIKI on bare gold 55 and f-MVPIKI on graphene-coated SPR sensor chips 54 without linkers. Figure 20f shows bar graphs of mean SPR signals 41 for peptide 1 interaction on bare gold 56 and graphene-coated SPR sensor chips 57 without linkers 50, 51. Statistical significance between groups was determined using Two-way ANOVA, n=3. Significance levels are indicated as follows: ns, P≥ .05; *, P< .05; **, P< .01; ***, P< .001.

For comparing the immobilization of the peptides 1 on gold and graphene, the peptides 1 were immobilized on the substrates 2 surface by the linker 5 molecules 4-mercaptobutyric acid (4-MBA) for gold as a gold linker 51 and 1-pyrenebutyric acid (PBA) for graphene as a graphene linker 50 (see Figure 20a). The linker 5 molecules have a similar length. The gold linker 51 4-mercaptobutyric acid (4-MBA) binds covalently to gold and the graphene linker 50 1-pyrenebutyric acid (PBA) binds to the graphene surface via π-π interactions. Since both peptides 1 contain a lysine (K), the butyric acid can bind the peptide using an amine coupling approach. Figure 20b shows sample traces corresponding to the immobilization of f-MVPIKI 52 on gold (blue curve 55) and graphene (red curve 54) using amine coupling, see f-MVPIKI on graphene 54 and f-MVPIKI on gold 55. The resulting SPR signal on graphene 54 is much higher than that on gold 55. Similar data were obtained for the peptide 1 f-MEQQNK 53.

Figure 20c summarizes the data and shows that a significantly higher response was measured on a graphene substrate 2 than on the gold substrate 2 for both peptides 1. This effect is more pronounced for f-MEQQNK 53 (6.1-fold) than for f-MVPIKI 52 (2.2-fold). In f-MEQQNK 53, lysine (K) is the last amino acid, which may improve its availability for binding to the linker molecules. For f-MVPIKI 52, lysine (K) is the second to last amino acid, which may decrease the availability for binding compared to the other peptide f-MEQQNK 53. Overall, this signal enhancement on graphene versus gold is consistent, suggesting a general positive effect associated with graphene coated with linker molecules.

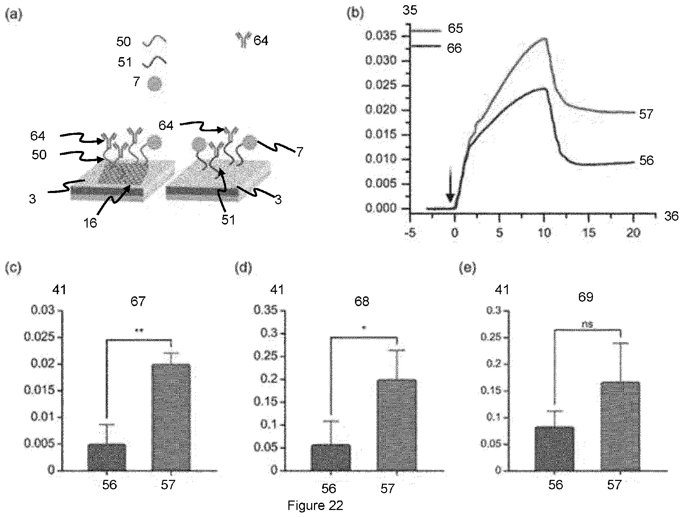

To investigate the positive effect of the linkers 51 on the quality of peptide 1 immobilization, linker control experiments were performed, see Figures 20d-f. Therein both peptides 1 were injected over bare gold 55 and graphene-coated chips 54 in the absence of linker molecules 50, 51 and corresponding amine chemistry as shown in schematic Figure 20d. The corresponding sensograms in Figure20e shows a high level of physical adsorption of f-MVPIKI on gold 55 compared to f-MVPIKI graphene 54 in the absence of linkers 51. Similar results were observed with f-MEQQNK 53, as can be seen in Figure 20f. The interaction of f-MVPIKI with graphene 54 is driven by hydrophobic forces, with electrostatic interactions also playing a role. The adsorption of hydrophobic peptides onto the graphene surface is stable and can be influenced by factors such as pH. The peptides 1 can also preferentially bind to the edges or planar surfaces of graphene, affecting its electronic properties. Due to its hydrophilic nature, f-MEQQNK 53 exhibits relatively low physical binding to the graphene surface, as shown in Figure 20f. Hence, hydrophobic peptides 1 tend to absorb onto gold surfaces 55 because they favorably interact with them. These interactions are driven by van der Waals forces and hydrophobic interactions, as well as possible chemical bonding between sulfur-containing amino acids and gold. Since both peptides 1 contain a sulfur group in the amino acid methionine, they bind more strongly to the gold surface than to the graphene surface in the absence of linkers 50, 51. Also, other mechanisms, such as electrostatic interactions, can facilitate their adsorption. This may explain why for f-MEQQNK 53 less binding in comparison to f-MVPIKI 52 to the gold surface is observed. Overall, the signal enhancement on graphene versus gold is consistent with the data obtained with antibodies (see Figure 22), demonstrates the positive effect associated with graphene coated with linker molecules 50, 51.

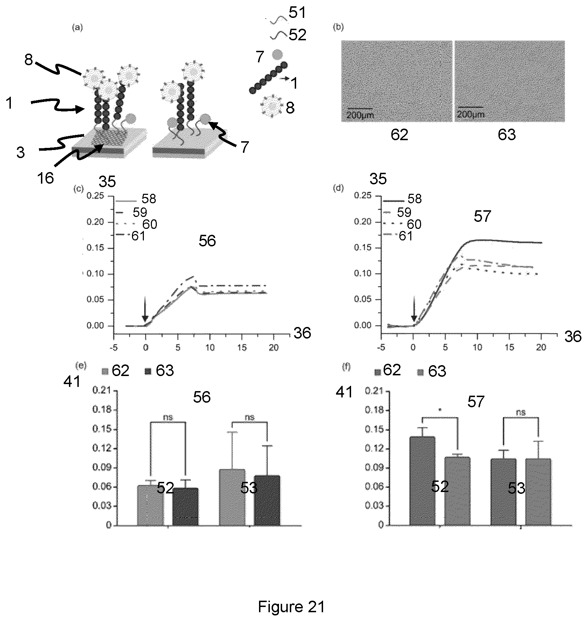

Assessing the levels of different types of immune cells 8 in human blood samples is an important test used to diagnose a wide range of medical conditions, from infections and allergies to certain types of cancer. It is advantageous to use peptide-coated substrates as sensor chips for discriminating between two relatively closely related immune cell types and investigating on how graphene or gold surfaces affected these measurements (see Figure 21a).

In Figure 21, peptide 1 and cell 8 interaction systems are shown. Figure 21a schematically shows peptide 1 immobilization and subsequent capture of THP1 cells 62 and HL60 cells 63 on graphene-coated substrates for SPR sensor chips functionalized with f-MVPIKI 52 or f-MEQQNK 53. Figure 21b shows microscopic images of THP1 cells 62 (left) and HL60 cells 63 (right). Figure 21c shows SPR sensograms showing responses of THP1 cells 62 and HL60 cells 63 flowing over gold SPR substrate 2 sensor chips functionalized with f-MVPIKI 52 or f-MEQQNK 53 at 30 µl/min. The following graphs are displayed: THP1 vs. f-MVPIKI 58, HL60 vs. f-MVPIKI 59, THP1 vs. f-MEQQNK 60 and HL60 vs. f-MEQQNK 61. The arrow marks the addition of the cells 8.

Figure 21d shows SPR sensograms showing responses of THP1 cells 62 and HL60 cells 63 flowing over graphene substrate 2 SPR sensor chips functionalized with f-MVPIKI 52 or f-MEQQNK 53 at 30 µl/min. Figure 21e shows bar graphs comparing interactions of THP1 cells 62 and HL60 cells 63 with gold substrate 2 chips functionalized with f-MVPIKI 52 or f-MEQQNK 53 measured on gold 56. Figure 21f shows bar graphs comparing interactions of THP1 cells 62 and HL60 cells 63 measured on graphene 57 substrate 2 chips functionalized with f-MVPIKI 52 or f-MEQQNK 53. Bars represent mean±SD, with n=3 for the measurements on gold 56 and n=4 for the measurements on graphene 57. Statistical significance between groups was determined using a regular two-way ANOVA. Significance levels are indicated as follows: ns, P≥.05; *, P<.05; **, P<.01.

Monocytes and neutrophils are the most common immune cell types in human blood samples. Therefore, leukemia cell lines THP1 62 and HL60 63 are investigated, which are similar to monocytes and neutrophils, respectively (see Figure 21b). Acute promyelocytic leukemia (APML) is a subtype of acute monocytic leukemia (AML) in which immature cells accumulate in the bone marrow, resulting in a reduced number of blood cells in the body. HL60 cells 63 are derived from APML patients, while THP1 cells 62 are derived from AML patients, but both cell types share similar expression patterns. Therefore, conventional methods fail to distinguish between the two cell types. Advantageously, the interaction of both cell types with gold and graphene substrates 2 that were functionalized with f-MVPIKI 52 and f-MEQQNK 53 peptides 1 was successful. Figure 21c shows a typical SPR sensogram illustrating the interaction of THP1 cells 62 and HL60 cells 63 with a gold substrate 2 sensor functionalized with f-MVPIKI 52 and f-MEQQNK 53. When a graphene monolayer is present on the substrate 2, the signals are generally higher (see Figure 21d). Moreover, there is a clear signal difference between THP1 cells 62 and HL60 cells 63 on graphene coated substrates 2 with f-MVPIKI 52. These measurements were repeated with several sensors, the results are summarized as bar graphs for gold (see Figure 21e) and graphene (see Figure 21f). Significant signal differences were observed between THP1 cells 62 and HL60 cells 63 on graphene-coated sensors, for f-MVPIKI 52. In all cases, the addition of graphene significantly increased the signal-to-noise ratio. In summary, these data show that peptide 1 coated substrates 2 surfaces in combination with graphene are highly advantageous biomedical tools for discriminating between different cell types. The observed differences between the peptides 1 are likely due to the different peptide 1 sequences, which by design differ in properties such as hydrophobicity, charge, the presence and position of certain amino acids.

**Table 1. Overview of results**

| Capture molecule | Target molecule | Enhancement factor (%) |
|---|---|---|
| 1-Pyrene butyric acid | Peptide (f-MVPIKI) | 300 |
| 1-Pyrene butyric acid | Peptide (f-MEQQNK) | 601 |
| f-MVPIKI | Cell (THP1) | 221 |
| f-MVPIKI | Cell (HL60) | 183 |
| f-MEQQNK | Cell (THP1) | 119 |
| f-MEQQNK | Cell (HL60) | 134 |

In summary, the addition of a monolayer of graphene to the gold surface of SPR chips significantly enhances signals by up to 600 times. The improved signal-to-noise ratio also allowed different cell types to be distinguished. The signal enhancement with graphene is attributed to improved plasmonic interaction and immobilization of molecules on the graphene surface. This graphene-enhanced plasmonic interface is versatile and adaptable for the study of diverse biochemical interactions in real time, providing a powerful tool for potential applications in biomedical research, diagnostics and therapy.

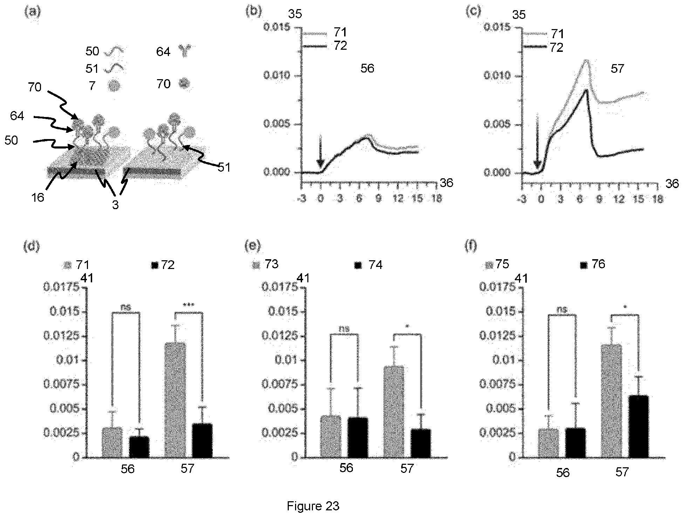

Figures 22 and 23 show the immobilization of antibodies 64 and related data. Figure 22 shows antibody 64 systems for virus 70 sensing (see Figure 23) on gold 56 and graphene 57 enhanced SPR sensors. Adeno-associated virus (AAV) was used as vector and is relevant for gene therapy. First, the chemical immobilization of three monoclonal antibodies A20 (reference sign 67), A20R (reference sign 69) and A20-h1 (reference sign 68) was demonstrated on gold 56 and graphene-coated 57 SPR sensors, which are gold coated glass slides 3 (schematic representation Figure 22a). The antibodies 64 were covalently attached by amine coupling to short linker molecules 50, 51 containing reactive butyric acid moieties (four carbon atoms), in particular similar as described before for the peptides 1. Two different linker molecules 50, 51 of similar length were used to modify gold and graphene: 4-mercaptobutyric acid (4-MBA), as a gold linker 51, which binds covalently to gold and 1-pyrenebutyric acid (PBA), as a graphene liner 50, which binds to the graphene surface via π-π interactions. After incubation with linker molecules 50, 51, an antibody 64 concentration of 8 µg/ml was injected into the SPR fluidic chamber. In Figure 22b the SPR sensogram shows successful immobilization of the A20 antibody on gold 66 and graphene 65, with a clear improvement in immobilization on the graphene sensor. As shown in Figure 22c-d, all three antibodies 64 achieved significantly higher immobilization on the graphene surface, with A20 67 showing the highest enhancement (4-fold) compared to the gold surface 66. The exact SPR signal enhancement achieved by these antibodies 64 on graphene compared to gold is described in table 2. This improved antibody 64 immobilization can be attributed to the higher density of binding sites and the higher adsorption capacity of graphene, especially a graphene monolayer.

**Table 2. Overview of Results**

| Capture molecule | Target molecule | Enhancement factor (%) |
|---|---|---|
| biotinylated BSA | Streptavidin | 37 |
| biotinylated cholera toxin (antigen) | Cholera Toxin (antibody) | 80 |
| 1-Pyrene butyric acid | Antibody (A20) | 400 |
| 1-Pyrene butyric acid | Antibody (A20-h1) | 348 |
| 1-Pyrene butyric acid | Antibody (A20R) | 201 |
| A20 | Virus (AAV2) | 373 |
| A20-h1 | Virus (AAV2) | 222 |
| A20R | Virus (AAV2) | 385 |

Figure 22 illustrates the immobilization of different antibodies 64 on gold 56 and graphene 57, in particular of an A20 antibody 67 (Figure 22c), an A20R antibody 69 (Figure 22e) and of an A20-h1 antibody 68 (Figure 22d). Figure 22a shows schematically the chemical attachment of antibodies 64 that differ in their Fc region to gold 56 and graphene-coated 57 SPR sensor chips. Figure 22b illustrates a SPR sensogram showing chemical bonding of the A20 antibody 67. Figure 22c-e show bar charts comparing immobilization of A20 67 in Figure 22c, A20-h1 68 in Figure 22d, and A20R 69 in Figure 22e on graphene 57 (red) and gold 56 (blue) chips, based on sensorgram data. Bars represent mean±SD, n=3. Statistical significance between groups was determined using independent samples t-test, n=3. Significance levels are indicated as follows: ns, P≥ .05; *, P< .05; **, P< .01; ***, P< .001.

All three antibodies 64 (A20, A20R and A20-h) were tested against AAV2, one of the most commonly used AAV serotypes in the gene therapy field, to compare pure gold 56 and graphene-coated 57 gold chips. It is important to note that all three antibodies 64 have different Fc regions, which may result in a different amounts of antibody 64 binding to the sensor surface. A20 67 is a mouse IgG3 monoclonal antibody that neutralizes AAV2 by binding to a conformational epitope on assembled capsids. A20-h1 68 is a recombinant human chimeric IgG1 antibody that can neutralize AAV2 and AAV3. A20R 69 is described as a recombinant mouse IgG1 antibody that specifically interacts with AAV2, AAV3 and Anc80 particles. AAV2 is specific to this family of antibodies and therefore expected to interact with all of them, while AAV8 is preferably used as a virus control and should show no affinity and reactivity to these monoclonal antibodies 64.

Figure 23a shows the schematic of a biosensing assay for AAV2 detection. The sensogram in Figure 23b shows a higher interaction of A20 with AAV2 71 on the graphene sensor. The results shown in Figure 23c-e confirm that the binding capacity of graphene sensors is superior to that of gold. The binding response between A20 and AAV2 67 on graphene 57 was 3.73 times higher than on gold 56, with measured values of 0.011 ± 0.005 RU on graphene 57 versus 0.003 ± 0.002 RU on gold 56 (Figure 23b). Similar results were observed for A20-h1 68 and A20R 69, where the SPR signal was 2.22 and 3.85 times higher on graphene, respectively.

Figure 21 illustrates a comparison of virus 70 particles capturing on gold and graphene sensors. Figure 23a shows a schematic representation of detection assays to capture AAV2 on gold and graphene enhanced SPR sensors while AAV8 is used as virus control. Figure 23b depicts SPR sensograms showing A20 vs. AAV2 71 (orange) and A20 vs. AAV8 72 (black) interaction to immobilize antibody A20 on gold chips 56, in particular at a flow rate of 20 µl/min. Figures 23d-f show bar charts comparing interactions of A20 vs. AAV2 71 and A20 vs. AAV8 72 (Figure 23d), of A20-h1 vs. AAV2 73 and A20-h1 vs AAV8 74 (Figure 23e), and A20R vs. AAV2 75 and A20R vs AAV8 76 (Figure 23f) functionalized graphene and gold sensors with AAV2 (orange) or control virus AAV8 (black), based on sensorgrams data. Bars represent mean±SD. Statistical significance between groups was determined using Two-way ANOVA, n=3. Significance levels are indicated as follows: ns, P≥ .05; *, P< .05; **, P< .01; ***, P< .001. The sensogram in Figure 23b shows lower binding when AAV2 and AAV8 were injected over A20. While the sensogram in Figure 23c shows a higher interaction signal of A20 with AAV2 on graphene sensor and smaller binding is observed when AAV8 (virus control) is injected over A20. The results shown in Figures 23d-f confirm the binding capacity of graphene sensors is superior to that of gold, simultaneously it also depicts that there is less interaction of all three antibodies 64 with control virus AAV8 on graphene. The antibody 64 interactions with AAV2 and AAV8 are statistically less significant on gold, the difference observed on graphene is statistically more significant, consistent with the lower signal at the detection limit (S_{dl}=0.0007) compared to gold (S_{dl}=0.0017) indicating enhanced sensitivity of the graphene-coated SPR sensor. The lower baseline drift of 1×10⁻⁴ RU/min also suggests the stability of these sensors.

In summary, all three antibodies 64 were able to interact significantly better on graphene sensors than on gold sensors. As shown in table 2, the graphene surface always gave more signal from the immobilized antibodies 64. For example, up to 3.85 times more signal on graphene than on gold for AAV2 detection agrees very well with up to 4 times more antibody 64 signal. This can be partly explained by the fact that the carboxyl group of PBA exhibits repulsive behavior towards the graphene surface and faces away from the sensor surface, improving the vertical orientation of the antibodies 64 on the graphene surface. This potentially increases the density and availability of antibody 64 binding sites for the virus 70 particles resulting in higher immobilization on graphene. This in turn suggests that AAV2 particles had a greater chance of interacting on graphene sensors compared to gold sensors.

The following materials may be used: 4-mercaptobutyric acid, 1-Pyrenebutyric acid, *N*-Hydroxysuccinimide (NHS), 1-Ethyl-3-(3-dimethylaminopropyl) carbodiimide (EDC) and Ethanolamine. Monolayer Graphene on polymer film. All chemicals utilized in the experiments were of analytical grade or higher quality. C1 assay buffer (130 mM NaCl, 10 mM HEPES, 5 mM KCI, 2 mM CaCl₂, 5 mM glucose, pH 7.4). Antibodies A20 (Cat. No. 690055), A20R (Cat. No. 690298) and A20-h1 (Cat. No. 692379) and AAV2 empty capsids (Cat. No. 66V020).

Multiparametric SPR experiments may be conducted using a MP-SPR device equipped with gold substrate 2 chips and flow cell with volume of 1µl/channel. The substrate 2 sensor slides, constructed of glass, were coated with a 2 nm layer of chromium and 50 nm layer of gold. SPR measurements were conducted in angular scan mode to monitor real-time changes in the position of the minimum SPR peak. The biomolecular interactions were analyzed at a wavelength of 670 nm. Before starting the SPR experiments, the sample injection system and flow channels were carefully washed and filled with a running buffer solution. Throughout the duration of the SPR measurement, the flow rate varied according to the step of the binding experiment, and the sensor temperature was maintained at 22 °C.

Graphene enhanced SPR chip preparation: The monolayer graphene sheet was transferred onto SPR substrate 2 chips using a wet transfer method. This monolayer graphene contained a thin layer of PMMA polymer film. The PMMA/graphene/gold substrate 2 chip was then air dried for 30 minutes. Afterwards it was cured at 150 °C for one hour and left under vacuum at room temperature overnight. By doing so the PMMA/graphene layer was fully in contact with the SPR substrate 2 chip. Now to remove the PMMA layer the chip was immersed in acetone and kept in the oven at 50 °C for one hour. After this the chip was immersed in isopropanol for one hour at room temperature. Now the chip was washed with acetone and blow-dried using nitrogen.

Functionalization of gold and graphene-enhanced SPR substrate 2 chips: The ligand peptide 1 was immobilized onto both gold and graphene-enhanced substrate 2 sensor surfaces using amine-coupling chemistry. For the gold substrate 2 chip, the bare substrate 2 sensor surface was first cleaned using oxygen plasma for 10 minutes, rinsed with ultrapure water, and dried under a nitrogen stream. The substrate 2 chip was then immersed in 4-mercaptobutyric acid for 60 minutes to functionalize the surface. In the case of the graphene-coated chip, the substrate 2 sensor surface was rinsed with ultrapure water, dried under a nitrogen stream, and then incubated in 1-pyrenebutyric acid for 60 minutes.

After these respective functionalization steps SPR substrate 2 chips were placed in SPR slide holders for the remaining experiments. An initial baseline was recorded in running buffer, followed by surface activation through a 7-minute injection of a 1:1 mixture of 0.1 M NHS and 0.4 M EDC at a flow rate of 20 µl/min. The ligand peptide (1 mM) or antibody (8µg/ml) was immobilized onto the activated substrate 2 surfaces of both chips. To block any unreacted esters by capping, a 7-minute injection of 1 M ethanolamine hydrochloride (pH 8.5) was applied. Finally, analytes such as living cells 8 (275k cells/ml) were flowed over the sensor surfaces at a constant flow rate for detection.

Cell culture: HL-60 and THP-1 cells 8 were cultured in RPMI-1640 media supplemented with fetal calf serum (10%), penicillin-streptomycin (1 unit/ml) and L-glutamine (2 mM) in cell culture suspension flasks. Cells were incubated at 37 °C and 5% CO₂ and were adjusted three times per week to a cell number of 300.000/ml to keep a suitable confluency. For experiments, cells 8 were taken from cell culture suspension, were centrifuged (300 g; 10 min) and subsequently resuspended to a cell number of 275.000/ml in C1 assay buffer (130 mM NaCl, 10 mM HEPES, 5 mM KCI, 2 mM CaCl₂, 5 mM glucose, pH 7.4). Cells were stored on ice until the start of the experiment to keep their viability.

MP-SPR Data Analysis: The MP-SPR Navi DataViewer^{™} software and TraceDrawer^{™} eases the extraction and analysis of data obtained from MP-SPR measurements, including the capture of cells on a peptide-modified sensor. After the completion of measurements, the software allows users to access and analyse the captured data. Theoretical analysis was done using LayerSolver^{™} software in simulation mode.

Statistical Analysis: All statistical analyses were carried out using BioRender R statistical software (version 4.2.2). For the analysis presented in Figure 22, the Shapiro-Wilk test was used to evaluate whether the data distributions for the "Gold" and "Graphene" groups significantly deviated from normality. To assess the equality of variances between the two groups, Levene's test was performed. Subsequently, an independent samples t-test, executed as a two-tailed test, was employed to compare the means of the "Gold" and "Graphene" groups. Furthermore, Cohen's d was calculated to measure the effect size, providing a standardized indication of the magnitude of the difference between the group means. Two-way analyses of variance (ANOVA) were employed to evaluate the effects of multiple independent variables on the dependent variable, namely the surface plasmon resonance (SPR) signal. As illustrated in Figure 23, the analysis evaluated the impact of surface type (Gold vs. Graphene) and virus 70 serotype (AAV2 vs. AAV8), thereby representing the interaction of A20, A20-h1, or A20R antibodies with each virus 70. As illustrated in Figure 20, the analysis investigated the impact of peptide sequence (f-MVPIKI and f-MEQQNK) and surface type (Gold and Graphene). Statistical analysis in Figure 21, the independent variables were two peptides (f-MVPIKI and f-MEQQNK) and cell type (THP1 and HL60). In all cases, the SPR signal was utilized as the dependent variable. For each analysis, statistically significant main effects and interaction effects were followed by post hoc pairwise comparisons using Bonferroni correction to adjust for multiple comparisons. The magnitude of the effects was calculated using partial eta-squared, which indicates the proportion of variance explained by each independent variable and their interaction. The statistical significance of each test was determined by the p-value, with a threshold of p < 0.05 being considered significant.

### Reference signs

- 1: peptide
- 2: substrate
- 3: gold coated slide
- 4: carboxylic acid
- 5: linker
- 6: activated succinimide
- 7: capping
- 8: cell
- 9: FP-receptor
- 10: detection zone
- 11: source electrode
- 12: drain electrode
- 13: gate electrode
- 14: fluidic system
- 15: detection device
- 16: graphene layer
- 17: gold layer
- 18: glass slide
- 20: light source
- 21: light beam
- 22: prism
- 23: plasmon wave
- 24: resonance angle
- 25: detector
- 26: field-effect transistor structure
- 27: plastic material
- 28: amperemeter
- 29: reference electrode
- 30: voltage source V_{DS}
- 31: voltage source V_{GS}
- 32: intensity
- 33: reference property
- 34: contact property
- 35: surface plasmon-polariton resonance signal
- 36: time
- 37: current
- 38: voltage
- 39: control sample
- 40: target sample
- 41: mean surface plasmon-polariton resonance signal
- 42: target HEK R⁺ cells
- 43: control HEK R⁺ cells
- 44: target HEK R⁻ cells
- 45: control HEK R⁻ cells
- 46: blocked target HEK R⁺ cells
- 47: blocked control HEK R⁺ cells
- 48: first donor
- 49: second donor
- 50: graphene linker
- 51: gold linker
- 52: f-MVPIKI
- 53: f-MEQQNK
- 54: f-MVPIKI on graphene
- 55: f-MVPIKI on gold
- 56: measurement on gold
- 57: measurement on graphene
- 58: THP1 vs. f-MVPIKI
- 59: HL60 vs. f-MVPIKI
- 60: THP1 vs. f-MEQQNK
- 61: HL60 vs. f-MEQQNK
- 62: THP1 cells
- 63: HL60 cells
- 64: antibody
- 65: A20 vs graphene
- 66: A20 vs gold
- 67: A20
- 68: A20-h1
- 69: A20R
- 70: Virus
- 71: A20 vs. AAV2
- 72: A20 vs AAV8
- 73: A20-h1 vs. AAV2
- 74: A20-h1 vs AAV8
- 75: A20R vs. AAV2
- 76: A20R vs AAV8

## Claims

1. Peptide (1) having an affinity for a FP-receptor (9) comprising a peptide consisting of the amino acid sequence represented by any of the SEQ ID NOS: 1 to 8, and/or
a peptide consisting of the amino acid sequence represented by any of the SEQ ID NOS: 1 to 8 in which at least one terminal amino acid is deleted, substituted or added or both terminal amino acids are deleted or substituted, and/or
a peptide consisting of the amino acid sequence represented by any of the SEQ ID NOS: 1 to 8 in which one internal amino acid is deleted, substituted or added.

2. Peptide (1) according to claim 1, wherein at least one amino acid of the amino acid sequence is connected to a fluorophore, preferably the amino acid connected to the fluorophore is lysine (K), more preferably the lysine (K) closest to the carboxyl terminus.

3. Substrate (2) for a detection device (15), in particular for a surface plasmon resonance spectrometer or a field-effect transistor, comprising a graphene monolayer (16) arranged on a surface, in particular a dielectric surface,
wherein the peptide (1) according to claim 1 or 2 is immobilized on the graphene monolayer (16) and/or
wherein an antibody (64) is immobilized on the graphene monolayer (16).

4. Substrate according to the previous claim for an optical sensor and/or an electrochemical sensor, in particular for a surface plasmon resonance spectrometer, comprising: a glass substrate, which is coated with a conductive layer, in particular with a gold layer, a silver layer, a chromium layer, and/or a platinum layer, wherein the conductive layer is coated with the graphene monolayer (16).

5. Substrate according to claim 3 for a field-effect transistor, comprising: a glass substrate and/or a polymer substrate, which is or which are coated with the graphene monolayer (16).

6. Substrate according to previous claims 3 to 5 comprising a cell (8) having a FP-receptor (9), wherein the cell (8) is immobilized on the substrate (2) via the peptide (1) having an affinity to the cells' (8) FP-receptor (9) and/or comprising a virus (70) having an affinity to the antibody (64), wherein the virus (70) is immobilized on the substrate (2) via the antibody (64).

7. Detection device (15) comprising
the substrate (2) according to any of the previous claims 3, 4 or 6, wherein the substrate (2) comprises at least one detection zone (10) with a detection surface formed on a conductive layer capable of supporting surface plasmon resonance,
an illumination system configured to incident radiation onto the detection surface (10) to excite surface plasmons,
a detection system configured to detect changes in the reflected and/or transmitted radiation resulting from interactions at the detection surface, and
preferably a fluidic system (14) configured to deliver a sample to the detection surface.

8. Detection device (15) comprising
the substrate (2) according to any of the previous claims 3, 5 or 6, wherein the substrate (2) comprises at least one detection zone (10) supporting the graphene monolayer (16),
a field-effect transistor structure (26) comprising a source electrode (11), a drain electrode (12) and a gate electrode (13),
wherein the graphene monolayer (16) extends between the source electrode (11) and the drain electrode (12), forming a conductive channel and wherein the gate electrode (13) is configured to modulate a conductivity of the conductive channel,
a detection system configured to detect changes in the electrical properties of the conductive channel, and
preferably a fluidic system (14) configured to deliver a sample to the detection zone.

9. Process for immobilizing the peptide (1) according to claim 1 or 2 to a substrate (2) comprising steps of:
providing the substrate (2), the peptide (1) and a linker (5) having an affinity for the substrate (2),
(a1) contacting the linker (5) to the substrate (2) for immobilizing the linker (5) on the substrate (2),
(a2) connecting the peptide (1) to the linker (5) for obtaining a peptide-linker conjugate and immobilizing the peptide (1) on the substrate (2),
or
(b1) connecting the peptide (1) to the linker (5) for obtaining a peptide-linker conjugate, and
(b2) contacting the peptide-linker conjugate with the substrate (2) for immobilizing the peptide on the substrate (2).

10. Process, in particular for detecting the presence of a cell (8) having a FP-receptor (9) and/or a virus (70) having an affinity for the antibody (64) comprising steps of:
providing a substrate (2), preferably according to any of the previous claims 3 to 6, equipped with the peptide (1) according to claim 1 or 2 and/or with an antibody (64) and
a sample comprising a plurality of cells (8), of which at least one cell has a FP-receptor (9), and/or viruses (70) of which at least one virus (70) has an affinity for the antibody (64),
measuring a reference property (33) in the absence of the sample,
contacting the sample with the substrate (2),
measuring a contact property (34) during and/or after contacting the substrate with the sample (2),
comparing the reference property (33) to the contact property (34) for determining the presence or absence of the at least one cell (8) having a FP-receptor (9), and/or the least one virus (70) having an affinity for the antibody (64).

11. Use of the peptide (1) according to claim 1 or 2 for immobilizing a cell (8) having a FP-receptor (9) to a substrate (2).

12. Use of the peptide (1) according to claim 1 or 2 in a detection device (15) for determining the presence or absence of at least one cell (8) having a FP-receptor (9) in a sample.

13. Polynucleotide encoding the peptide (1) of claim 1.

14. Expression vector for expressing the peptide (1) according to claim 1.

15. Peptide-linker conjugate comprising the peptide (1) according to claim 1 or 2 and a linker (5) having an affinity for a graphene substrate (16) or a gold substrate (17), wherein the peptide (1) is connected to the linker (5).
